# EUROPÄISCHE PATENTSCHRIFT

(11) **EP 1 761 219 B1**
(45) Veröffentlichungstag und Bekanntmachung des Hinweises auf die Patenterteilung: **24.07.2013**
(21) Anmeldenummer: 05769201.4
(22) Anmeldetag: 28.06.2005
(51) Int. Cl.: A61F 2/66

(54) **KÜNSTLICHER FUSS**
ARTIFICIAL FOOT
PIED ARTIFICIEL

(30) Priorität: 29.06.2004 DE 10431562
(43) Veröffentlichungstag der Anmeldung: 14.03.2007
(73) Patentinhaber: Otto Bock HealthCare GmbH, 37115 Duderstadt (DE)
(72) Erfinder: MOSLER, Lüder, 37115 Duderstadt (DE)
(74) Vertreter: Lins, Edgar
(86) Internationale Anmeldenummer: PCT/DE2005/001156
(87) Internationale Veröffentlichungsnummer: WO 2006/000211

(56) Entgegenhaltungen:
- DE-A1- 10 010 302
- DE-C- 303 735
- DE-C- 309 066
- FR-A- 528 796
- US-A- 2 475 373
- US-A- 5 913 902
- US-A1- 2003 045 944

## Beschreibung

Die Erfindung betrifft einen künstlichen Fuß mit einem Anschlussteil für ein Unterschenkelteil, einem mit dem Anschlussteil über ein Knöchelgelenk drehbar verbundenen Basis-Fußteil, das sich posterior vom Knöchelgelenk in einen Fersenabschnitt und anterior vom Knöchelgelenk in einen Mittelfußabschnitt erstreckt, und mit einem mit dem Mittelfußabschnitt drehbar verbundenen Vorfußteil.

Ein derartiger künstlicher Fuß ist durch US 5,913,902 bekannt. Das Knöchelgelenk befindet sich dabei in der Achse eines von einem Adapteransatz des Anschlussteils aufgenommenen rohrförmigen Unterschenkelteils einer Unterschenkelprothese. Das Knöchelgelenk und das Gelenk zwischen Vorfußteil und Basis-Fußteil sind unabhängig voneinander drehbar, wobei die Drehbarkeit durch zwischen den jeweiligen Gelenkteilen eingesetzte Druckfedern elastisch gedämpft ist. Diese Fußkonstruktion erlaubt die Verwendung des künstlichen Fußes mit mehreren Absatzhöhen, da sich der Fuß auf verschiedene Winkel des Vorfußes und des Anschlussteils relativ zum Basis-Fußteil einstellen kann. Allerdings verändert sich dabei der statische Aufbau in Abhängigkeit von der Fersenhöhe, sodass auch stark unterschiedliche Gangdynamiken in Abhängigkeit von der Fersenhöhe entstehen.

DE 100 10 302 A1 offenbart einen Prothesenfuß mit einem beweglichen Knöchelgelenk und einem ebenfalls beweglichen Vorfußteil. Ziel des offenbarten Fußaufbaus ist es, ein Absinken des Kniegelenks - und damit des Hüftgelenks - beim Abrollen über die Fußspitze weitgehend zu vermeiden. Hierzu wird eine Plantarflexion zwischen Fuß und Unterschenkel, also zwischen Basis-Fußteil und Anschlussteil, angestrebt. Zu diesem Zweck ist eine Koppelstange zwischen dem Vorfußteil und dem Basis-Fußteil befestigt, wobei die Befestigung in dem Basis-Fußteil mit einem Langloch erfolgt. Ein Ende der Koppelstange ragt in den Bereich des Anschlussteils und begrenzt aufgrund der Beugung des Vorfußteils den Winkel zwischen Anschlussteil und Basis-Fußteil. Durch die Begrenzung des Winkels wird das weitere Absenken des Knies - und damit der Hüfte - vermieden. Eine Anpassung des Fußes an verschiedene Absatzhöhen ist mit der beschriebenen Konstruktion weder vorgesehen noch möglich.

FR 528 796 beschreibt einen künstlichen Fuß, dessen Anschlussteil über ein Kardangelenk mit einem Basis-Fußteil verbunden ist. Ein Vorfußteil ist schwenkbar an das Basis-Fußteil im Sohlenbereich angelenkt und mit dem Kardangelenk über einen Koppellenker verbunden. Eine Bewegung des Anschlussteils aus der Ausgangsstellung im Stand nach posterior führt dabei zu einem Absenken des Vorfußteils, eine Bewegung des Anschlussteils nach anterior zu einem Anheben des Vorfußtells. Die Verwendung eines derartigen künstlichen Fußes mit einem erhöhten Absatz würde daher zu einem Absenken des Vorfußteils führen, woraus ein unsicherer Stand resultieren würde. Der künstliche Fuß ist daher für die Verwendung mit unterschiedlichen Absatzhöhen weder vorgesehen noch geeignet.

Es sind Prothesen bekannt, bei denen die sagittale Einstellung des Prothesenfußes durch den Patienten erfolgen kann, um eine Anpassung an unterschiedliche Absatzhöhen vorzunehmen. Eine Falscheinstellung ist dabei nicht ausgeschlossen. Damit sich der Vorfuß an die Schuhform anpassen kann, ist dieser weich ausgeführt. Hierdurch wird jedoch eine ausreichende Lastaufnahme im Vorfußbereich am Ende der Standphase verhindert.

Der vorliegenden Erfindung liegt die Aufgabe zugrunde, einen künstlichen Fuß der eingangs erwähnten Art so auszubilden, dass er sich an unterschiedliche Absatzhöhen selbsttätig anpassen kann und dabei eine gute Standsicherheit gewährleistet.

Die Lösung dieser Aufgabe gelingt bei einem künstlichen Fuß der eingangs erwähnten Art erfindungsgemäß durch eine Gelenkverbindung, die eine Winkelstellung des Anschlussteils relativ zum Basis-Fußteil proportional auf die Winkelstellung des Vorfußteils relativ zum Basis-Fußteil derart überträgt, dass eine Veränderung der Winkelstellung des Anschlussteils nach posterior zu einem proportionalen Anheben des Vorfußteils führt.

Der erfindungsgemäße Fuß sieht somit eine Zwangskopplung zwischen dem Anschlussteil und dem Vorfußteil bezüglich der Winkelstellung relativ zum Basis-Fußteil vor. Gemäß dieser Zwangskopplung führt eine Veränderung der Winkelstellung des Anschlussteils nach posterior zu einem proportionalen Anheben des Vorfußteils. Wird der künstliche Fuß von einer Stellung ohne Absatz ("Barfuß-Stellung") in einen Schuh mit einem hohen Absatz eingeführt, ändert sich die Stellung des Unterschenkels (und damit des Anschlussteils) relativ zum Basls-Fußteil im Sinne einer Verstellung des Winkels nach posterior. Demzufolge wird das Vorfußteil soweit angehoben, dass es wieder parallel zur Auflagefläche (vordere Sohle des Schuhs) liegt. Es ist daher nicht erforderlich, das Vorfußteil weich auszugestalten, sodass das Vorfußteil fest auf der Standfläche aufliegt und somit die benötigte Last für ein sicheres Stehen, auch mit dem hohen Absatz, aufnimmt.

Die Gelenkanordnung ist dabei eine Mehrgelenk-Anordnung und kann insbesondere eine Viergelenk-Anordnung sein.

Die Gelenkverbindung ist vorzugsweise mit dem Mittelfußabschnitt und mit einem mit Vorfußteil und Anschlussteil gelenkig verbundenen Lenker gebildet, die dann in einer Viergelenk- oder Mehrgelenk-Anordnung angeordnet sind, wobei Mittelfußabschnitt und Lenker im Wesentlichen parallel zueinander liegen können.

In einer bevorzugten Ausführungsform der Erfindung ist das Anschlussteil mit einem sich über das Knöchelgelenk nach unten erstreckenden Ansatz versehen, an dem der Lenker mit einem seiner freien Enden drehbar befestigt ist.

Die erfindungsgemäße Gelenkverbindung kann durch Drehgelenke, aber auch durch Materialscharniere gebildet sein, wobei die Materialscharniere durch einen flexiblen Abschnitt eines Koppelstücks der Gelenkverbindung gebildet sind.

Die Funktion des erfindungsgemäßen künstlichen Fußes beruht darauf, dass das Basis-Fußteil als eine Wippe ausgebildet ist, die etwa mittig zwischen Ferse und Ballenbereich gelagert ist. Der Prothesenfuß generiert damit einen Aufbau, der die Lastlinie stets durch den Lagerpunkt der Wippe verlaufen lässt. Dies entspricht den Gegebenheiten beim natürlichen Fuß. Aufgrund der Kopplung des Anschlussteils und des Vorfußteils verläuft der Vorfuß immer etwa parallel zum Boden. Dies entspricht der Ausbildung von Schuhen mit einer konstanten Sohlendicke in diesem Bereich, was notwendig ist, da andernfalls der Fuß in die Schuhspitze gedrückt werden würde.

Wenn der erfindungsgemäße Fuß nur mit starren Elementen aufgebaut wird, baut er in der mittleren Standphase sehr schnell einen Vorfuß-Widerstand auf, der dem Standgefühl mit einem natürlichen Fuß nicht entspricht. Zur Anpassung an das natürliche Standgefühl ist es vorteilhaft, wenigstens ein Koppelstück der Gelenkanordnung in Längsrichtung elastisch verformbar auszubilden. Besonders zweckmäßig ist dies für ein Koppelstück, das in der Gelenkanordnung vorwiegend auf Zug belastet ist. In diesem Fall kann das Koppelstück als gekrümmte Blattfeder ausgebildet sein, die auf Zug zunächst weich anspricht und dann progressiv die Steifigkeit erhöht.

Weitere Verbesserungsmöglichkeiten ergeben sich bezüglich der Gangdynamik. Beim Fersenauftritt des Gehvorganges kippt das erfindungsgemäß als Wippe ausgebildete Basis-Fußteil nach vom, wodurch das Anschlussteil relativ nach hinten geneigt und somit das Vorfußteil nach oben gezogen wird. Will man diesen Effekt vermeiden, muss zwischen einer kurzfristigen starken Belastung des Fersenabschnitts des Basis-Fußteils bei dem Fersenauftritt beim Gehen und der längerfristigen Belastung für die Adaptierung der Absatzhöhe unterschieden werden. Dies kann dadurch erfolgen, dass ein Einstellelement für den Winkel zwischen Basis-Fußteil und Anschlussteil vorgesehen wird, das erst bei einer über mehrere Sekunden andauernden Belastung wirksam wird. Ein derartiges Einstellelement kann ein Hydraulikzylinder sein. Dieser wird zweckmäßigerweise mit einem durch kurzzeitige Belastungen (beim Fersenauftritt) verformbaren elastischen Glied kombiniert, um eine abfedernde Wirkung beim Fersenauftritt während des Gehens zu erzielen.

Die Unterscheidung zwischen kurzzeitiger Belastung beim Fersenauftritt und langfristige Anpassung der Absatzhöhe kann vorzugsweise dadurch erfolgen, dass in einen Umströmungsweg des Hydraulikzylinders eine Ventilanordnung eingefügt ist, die die Strömung der Hydraulikflüssigkeit in dem Umströmungsweg unterbricht, wenn die Strömungsgeschwindigkeit plötzlich ansteigt. Bei einer langsamen Anpassung sperrt die Ventilanordnung hingegen nicht, sodass die Hydraulikflüssigkeit für die Veränderung des durch den Hydraulikzylinder gebildeten Einstellelements fließen kann.

Beim Gehvorgang rollt der Fuß in üblicher Weise über den Vorfußbereich ab. Dabei wird der Unterschenkel (das Anschlussteil) relativ zum Basis-Fußteil nach vorn gekippt, sodass es durch die erfindungsgemäße Kopplung zu einem Herunterdrücken des Vorfußteils kommt, der Vorfuß somit nach unten "einkrallt". Dies lässt sich durch eine Ausgestaltung der Gelenkverbindung abmildern, die einen verminderten Übertragungsfaktor bezüglich der Winkelstellungen bewirkt. Dadurch wird aber auch die Anpassung an die unterschiedlichen Absatzhöhen beeinträchtigt.

Bevorzugt ist daher, dass wenigstens ein Koppelstück der Gelenkanordnung längenverstellbar ausgebildet ist, wobei die Längenverstellung durch wenigstens einen Sensor steuerbar ist, der Parameter der Geh- oder Stehsituation detektiert. Die Längenverstellung kann durch einen Hydraulikzylinder ausgeführt sein.

Zweckmäßig ist in jedem Fall, ein elastisches Glied zwischen Vorfußteil und Basis-Fußteil anzuordnen, das insbesondere die Längenverstellung eines Koppelstücks, insbesondere des Lenkers, ermöglicht.

Die elastische Ausbildung des Lenkers kann ergänzt oder ersetzt werden durch eine elastische Ausbildung des Ansatzes des Anschlussteils, an dem der Lenker angelenkt ist. Dabei kann der Ansatz selbst aus einem elastischen Material gebildet sein oder elastisch mit dem übrigen Rumpf des Anschlussteils schwenkbar verbunden sein.

Besonders zweckmäßig ist es für die vorliegende Erfindung, wenn das Knöchelgelenk mit Abstand anterior von einem das Unterschenkelteil aufnehmenden Adapteransatz des Anschlussteils angeordnet ist. Hierdurch wird eine gute Standsicherheit erreicht und werden die benötigten Drehmomente für die Steuerung des Fußes aufgebracht.

Die Erfindung soll im Folgenden anhand von in der Zeichnung dargestellten Ausführungsbeispielen näher erläutert werden. Es zeigen:
- Figur 1: einen prinzipiellen Aufbau eines künstlichen Fußes gemäß einer ersten Ausführungsform, dargestellt in einer Barfuß-Stellung;
- Figur 2: eine prinzipielle Darstellung des Fußes gemäß Figur 1 in einem Schuh mit einem hohen Absatz;
- Figur 3: eine Darstellung gemäß Figur 1 mit einer Verdeutlichung der Lage des Knöchelgelenks;
- Figur 4: eine Darstellung gemäß Figur 3 für eine nach vom verlagerte Belastung im Stand;
- Figur 5: eine Darstellung des Fußes gemäß Figur 1 während des Gehens bei einem Fersenauftritt;
- Figur 6: eine prinzipielle Darstellung einer Variante des Fußes gemäß Figur 1 entsprechend einem zweiten Ausführungsbeispiel;
- Figur 7: eine schematische Darstellung einer möglichen Wirkungsweise eines Dämpfers;
- Figur 8: eine dritte Ausführungsform eines erfindungsgemäßen Fußes mit einem elastischen Lenker im Ruhezustand;
- Figur 9: eine Darstellung gemäß Figur 8 für eine Abrollbewegung über den Vorfuß;
- Figur 10: eine vierte Ausführungsform des erfindungsgemäßen Fußes mit einem längenverstellbaren Lenker;
- Figur 11: eine fünfte Ausführungsform als konstruktive Variante zu der Ausführungsform gemäß Figur 10;
- Figur 12: eine sechste Ausführungsform mit einer Gelenkanordnung mit einem Gelenk nach Art eines Schamiergelenks.

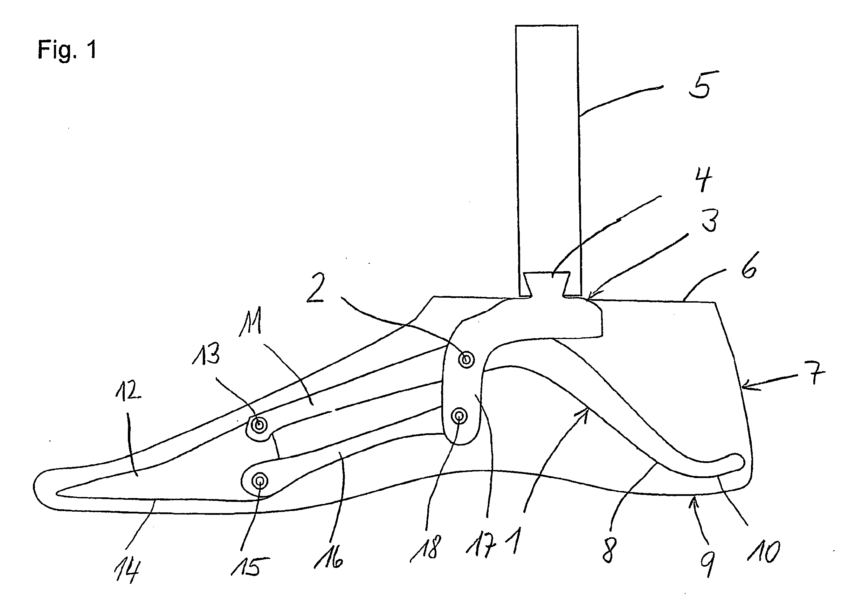

Eine erste Ausführungsform eines erfindungsgemäßen Fußes ist in den Figuren 1 bis 5 in einem prinzipiellen Aufbau dargestellt. Der funktionale Aufbau des Fußes enthält ein Basis-Fußteil 1, das über ein als Drehgelenk ausgebildetes Knöchelgelenk 2 mit einem Anschlussteil 3 verbunden ist. Das Anschlussteil 3 weist einen Adapteransatz 4 in Form eines auf dem Kopf stehenden Kegelstumpfes auf, der zur justierbaren Aufnahme eines rohrförmigen Unterschenkelteils 5 einer Unterschenkelprothese dient. Der Adapteransatz 4 ragt somit aus einer oberen Begrenzungsfläche 6 eines kosmetischen Überzugs 7 der Fußprothese heraus, die die funktionellen Teile der Fußprothese bis zur Abschlussfläche 6 umschließt.

Das Basis-Fußteil 1 bildet posterior von dem Knöchelgelenk 2 einen Fersenabschnitt 8 aus, der sich von dem Anschlussteil 3 schräg nach hinten unten erstreckt und in einer der Sohle 9 des kosmetischen Überzugs 7 nahen Fersen-Auflagefläche 10 endet. Nach anterior erstreckt sich das Basis-Fußteil 1 von dem Knöchelgelenk 2 in einem im Wesentlichen geradlinigen Mittelfußabschnitt 11, an dessen vorderem Ende ein den Zehenbereich repräsentierendes Vorfußteil 12 an einem Drehgelenk 13 angelenkt ist. Das Vorfußteil 12 bildet einen etwa dreieckförmigen Keil aus, dessen Unterseite 14 parallel zur Sohle 9 des kosmetischen Überzugs 7 im Bereich des Vorfußes 12 verläuft. Das Drehgelenk 13 befindet sich an der oberen Spitze des dreieckförmigen Vorfußteils 12. Im unteren Bereich des dreieckförmigen Vorfußteils 12 befindet sich ein weiteres Drehgelenk 15, über das das Vorfußteil 12 mittels eines Lenkers 16 mit dem Anschlussteil 3 verbunden ist. Hierzu weist das Anschlussteil 3 einen nach unten ragenden starren Ansatz 17 auf, an dem sich ein Drehgelenk 18 für die Befestigung des Lenkers 16 befindet. Das Anschlussteil 3 ist somit L-förmig mit einem etwa parallel zur Abschlussfläche 6 liegenden horizontalen Schenkel und einem etwa rechtwinkelig dazu nach unten ragenden Schenkel in Form des Ansatzes 17 ausgebildet.

Die Achsen der Drehgelenke 2, 13, 15, 18 liegen parallel zueinander und quer zur Sagittalebene des Fußes, also parallel zur Frontalebene des Patienten. In der dargestellten ersten Ausführungsform sind die Abstände zwischen den Drehgelenken 13, 15 einerseits und 2, 18 andererseits etwa gleich, sodass der Mittelfußabschnitt 11 und der Lenker 16 etwa parallel zueinander ausgerichtet sind (im Hinblick auf die Verbindungslinie zwischen den Drehgelenken 2, 13 einerseits und 15, 18 andererseits). In der dargestellten Ausführungsform entspricht die Gelenkverbindung zwischen dem Anschlussteil 3 und dem Vorfußteil 12 somit einer Parallelogrammführung.

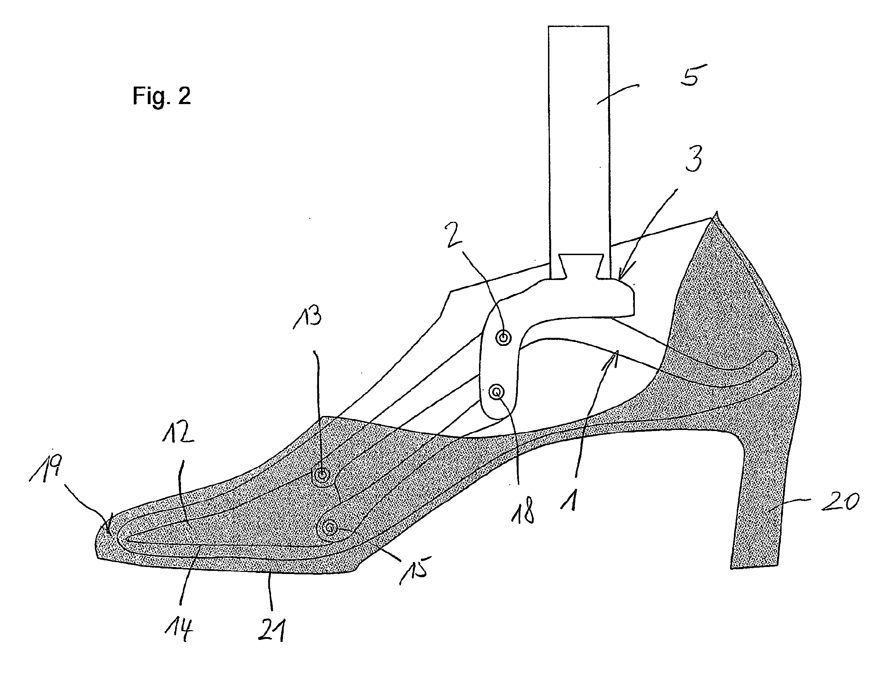

In Figur 1 ist der dargestellte künstliche Fuß in einer Barfuß-Stellung, d. h. ohne Absatz, dargestellt. Im Vergleich hierzu zeigt Figur 2 den Fuß gemäß Figur 1 in einem Schuh 19, der einen hohen Absatz 20 aufweist. Demgemäß ist das Anschlussteil 3 bzw. das Unterschenkelteil 5 relativ zu dem Basis-Fußteil 1 nach hinten gekippt, wodurch über die Gelenkverbindung mit den Gelenken 2, 13, 15, 18 das Vorfußteil 12 relativ zum Basis-Fußteil 1 nach oben geschwenkt wird. Da das Basis-Fußteil 1 in dem Schuh 19 wegen des hohen Absatzes 20 schräg nach vom abwärts gerichtet ist, wird die Verschwenkung des Vorfußteils 12 nach oben so eingestellt, dass die Unterseite 14 des Vorfußteils 12 parallel zur Sohle 21 des Schuhs 19 verläuft. Die dargestellte Gelenkverbindung sorgt somit für eine selbsttätige Anpassung des Fußes an die Höhe des Absatzes 20 mit Hilfe der Verschwenkung des Vorfußteils 12 mittels der Gelenkverbindung an den Drehgelenken 2, 13, 15, 18.

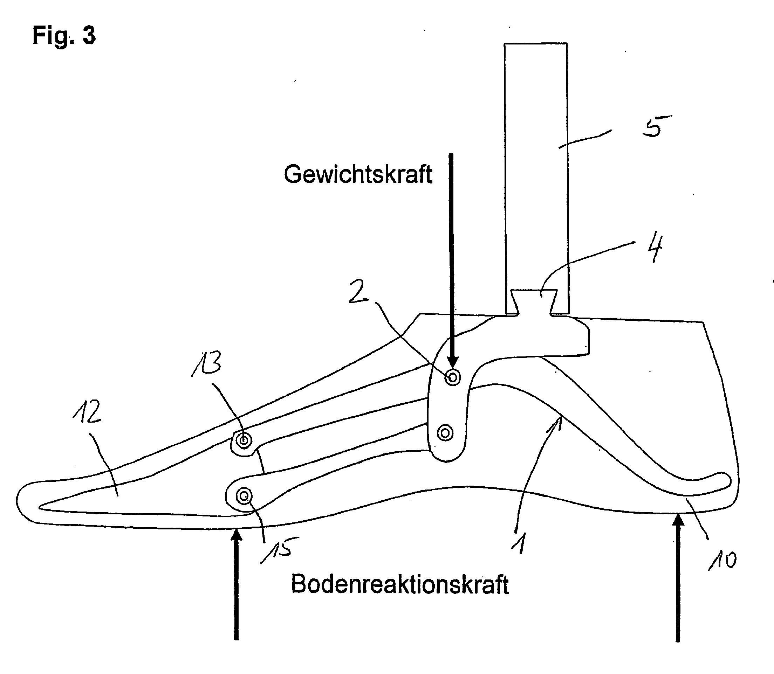

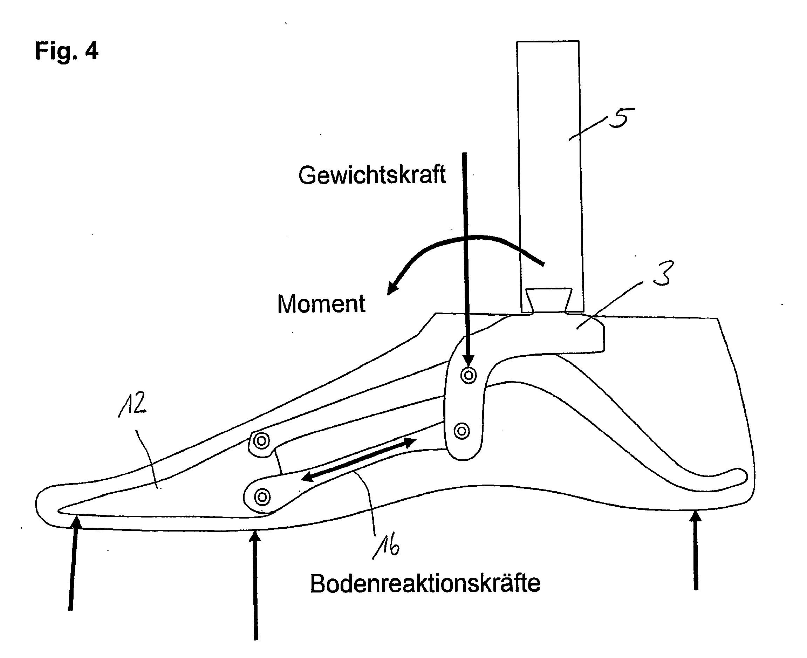

Die Figuren 3 und 4 verdeutlichen die vom erfindungsgemäßen Fuß aufgenommenen Kräfte. Die Gewichtskraft wird direkt in das Knöchelgelenk 2 eingeleitet. Das Knöchelgelenk 2 ist mit Abstand anterior von dem Adapteransatz 4 bzw. der Unterschenkelprothese 5 positioniert, wodurch das Knöchelgelenk 2 etwa mittig an dem als Wippe an dem Knöchelgelenk 2 angelenkten Basis-Fußteil 1 angreift. Die Bodenreaktionskräfte werden im Bereich der Fersenauflage 10 und im Ballenbereich, also etwa in Höhe der Drehgelenke 13, 15 des Vorfußteils 12 eingeleitet. Demzufolge befinden sich die Bodenreaktionskräfte etwa symmetrisch beiderseits der eingeleiteten Gewichtskraft, wenn ein stabiler Stand im Gleichgewicht ausgeübt wird.

Findet nun gemäß Figur 4 eine Gewichtsverlagerung nach vom statt, entsteht ein Drehmoment an dem Unterschenkelteil 5 bzw. Anschlussteil 3 in Richtung des in Figur 4 gekrümmt eingezeichneten Pfeils. Hierdurch wird an dem Lenker 16 eine Zugkraft nach posterior ausgeübt, wodurch die anteriore Spitze des Vorfußteils 12 nach unten gedrückt wird. Die Spitze ist daher in der Lage, zusätzliche Bodenreaktionskräfte aufzunehmen, wie dies durch den zusätzlichen Pfeil im Bereich der Spitze des Vorfußteils 12 in Figur 4 dargestellt ist. Die auf die Fersen-Auflagefläche 10 einwirkende Bodenreaktionskraft hat sich in diesem Fall durch die Gewichtsverlagerung nach vorn verringert.

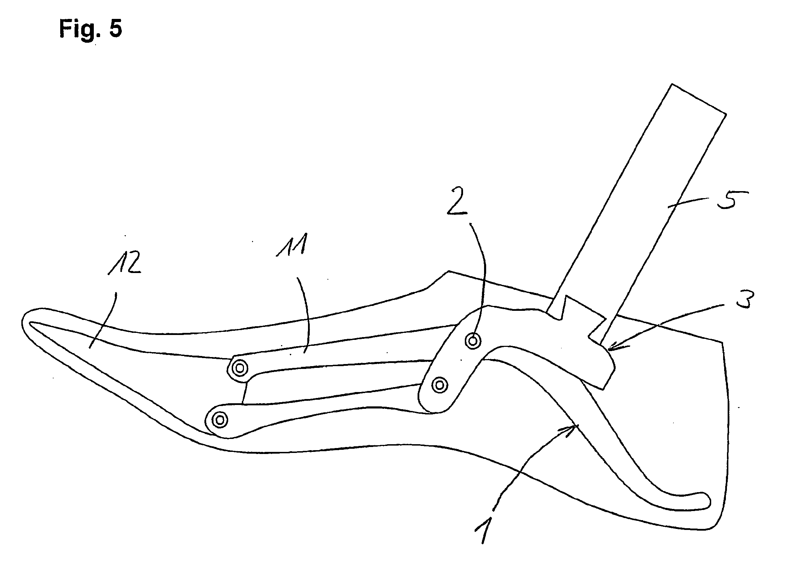

Figur 5 zeigt den Fuß gemäß Figur 1 in einer Fersenauftrittsphase beim Gehen. In diesem Fall ist das Unterschenkelteil 5 bzw. das Anschlussteil 3 gegenüber dem Basis-Fußteil 1 nach hinten gekippt, sodass das Vorfußteil 12 anatomisch richtig etwas noch oben verschwenkt ist. Beim Aufsetzen des Fußes im Bereich der Ferse verschwenkt die das Basis-Fußteil 1 bildende Wippe um das Knöchelgelenk 2 schlagartig mit dem Mittelfußabschnitt 11 nach unten, sodass der nach hinten verschwenkte Winkel des Unterschenkelteils 5 relativ zum Basis-Fußteil 1 noch vergrößert wird, wodurch das Vorfußteil 12 extrem nach oben verschwenkt wird.

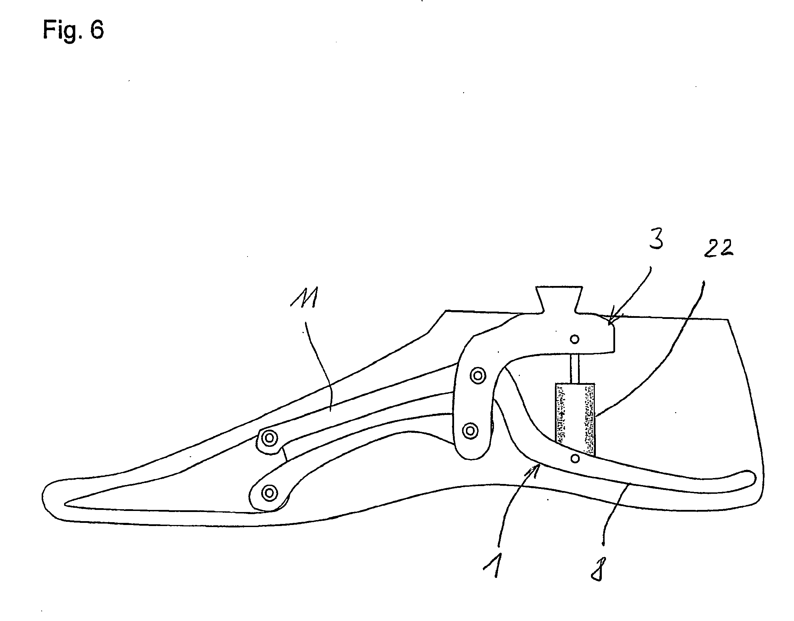

Es kann daher zweckmäßig sein, gemäß Figur 6 die Verschwenkung des Basis-Fußteils 1 beim Fersenauftritt dadurch zu dämpfen, dass zwischen dem Fersenabschnitt 8 des Basis-Fußteils 1 und dem Anschlussteil 3 ein Dämpfer 22, beispielsweise in Form eines Hydraulikdämpfers eingesetzt ist. Der elastische Dämpfer verhindert ein ungedämpftes Herunterklappen des Mittelfußabschnitts 11 beim Fersenauftritt.

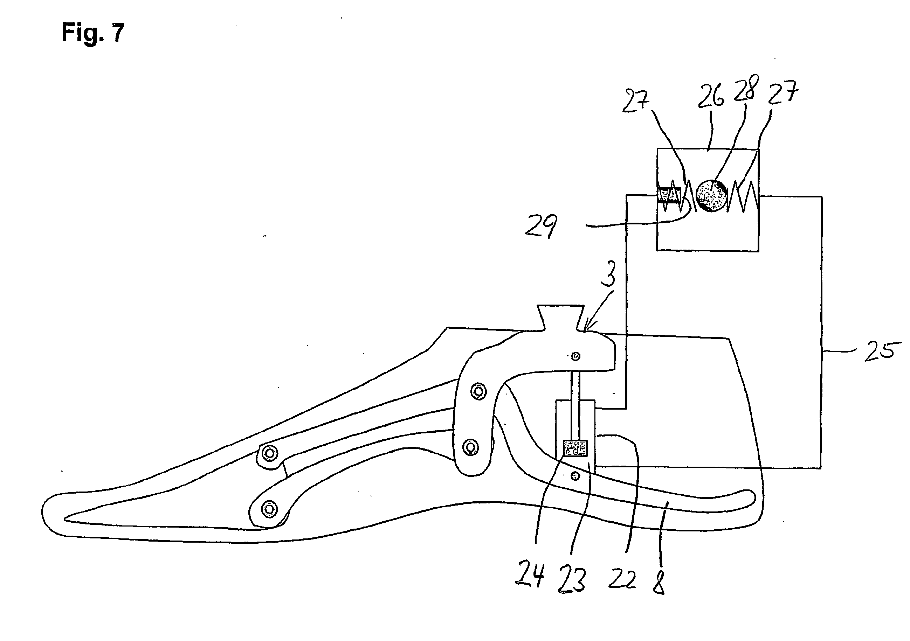

Figur 7 verdeutlicht eine bevorzugte Wirkungsweise des Dämpfers 22. Dieser besteht aus einem Zylinderraum 23 und einem Kolben 24. Die relative Bewegung zwischen Fersenabschnitt 8 und Anschlussteil 3 wird durch eine Umströmungsleitung 25 gedämpft, durch die die Hydraulikflüssigkeit von einer Seite auf die andere Seite des Kolbens 24 strömen muss. Vorzugsweise ist in die Umströmungsleitung 25 eine Ventilanordnung 26 eingesetzt, die - schematisch dargestellt - aus einer mit Federn 27 gehaltenen Absperrkugel 28 besteht. Bei einer geringen Strömungsgeschwindigkeit der Hydraulikflüssigkeit in der Umströmungsleitung 25, wie sie beispielsweise in einem quasi-statischen Zustand für die Anpassung an die unterschiedlichen Höhen eines Absatzes 20 auftritt, findet der erforderliche Abstandsausgleich mittels der Hydraulikflüssigkeit in gedämpfter Form statt.

Bei einer kurzzeitigen Belastungsspitze, wie sie beim Fersenauftritt während eines Gehvorganges entsteht, wird die Kugel 28 durch die hohe Strömungsgeschwindigkeit gegen eine Absperrsitz 29 gedrückt und versperrt den Strömungsweg für die Hydraulikflüssigkeit. In diesem Fall führt der Fersenauftritt nicht zu einer Änderung der durch den Hydraulikzylinder 22 bewirkten Abstandseinstellung zwischen dem Fersenabschnitt 8 und dem Anschlussteil 3.

Um einen elastischen Fersenauftritt zu ermöglichen, ist es zweckmäßig, in Serie zu dem Dämpfer 22 ein elastisches Glied, beispielsweise eine Feder, einzuschalten.

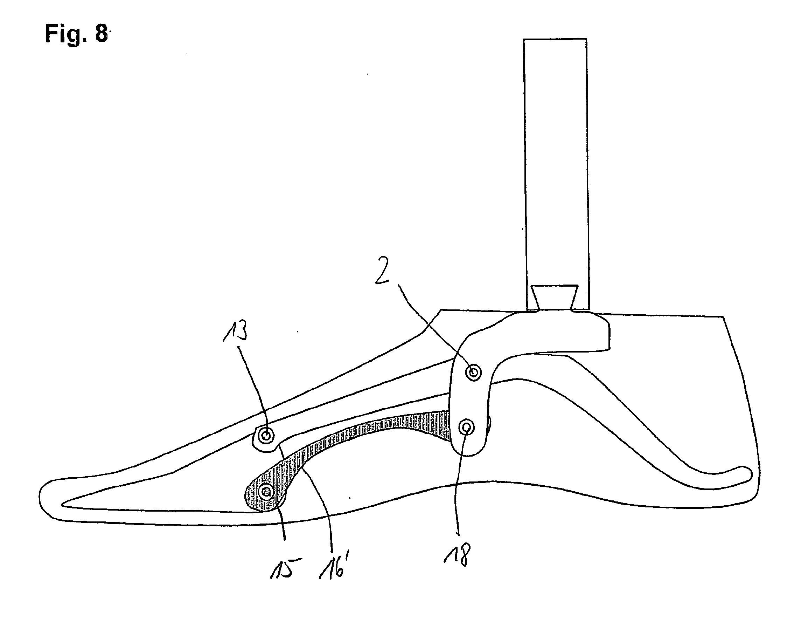

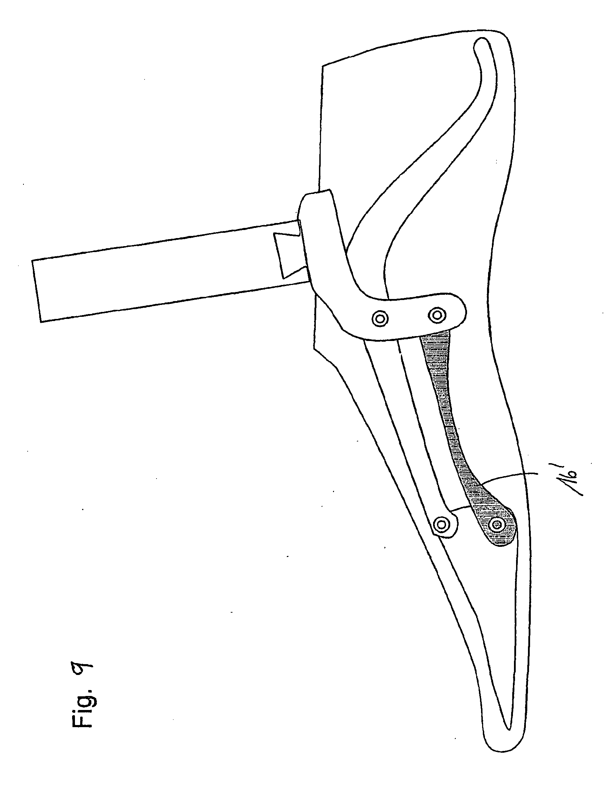

Die in den Figuren 8 und 9 dargestellte Ausführungsform weist eine gleiche Anordnung der Drehgelenke 2, 13, 15, 18 auf, allerdings mit der Variante, dass der Lenker 16' zwischen den Drehgelenken 15 und 18 durch eine gekrümmte Blattfeder gebildet ist. Figur 8 zeigt den Zustand bei einer Standbelastung im Gleichgewicht. Bei einer Vorverlagerung des Gewichts würde der starre Lenker 16 gemäß Figur 1 eine sofortige starke Vorfußbelastung hervorrufen, die nicht dem natürlichen Standgefühl entsprechen würde. Der Lenker 16' kann sich bei einer Vorverlagerung des Gewichts gemäß Figur 9 hingegen etwas elastisch längen, um dann bei einer weiteren Zugkraft auf den Lenker 16' einen progressiv anwachsende Vorfuß-Gegenkraft zu bewirken. Ein gewisses "Schwanken" beim Stehen des Patienten wird somit mit einer elastischen, progressiv anwachsenden Gegenkraft stabilisiert, was einem natürlichen Stehgefühl entspricht.

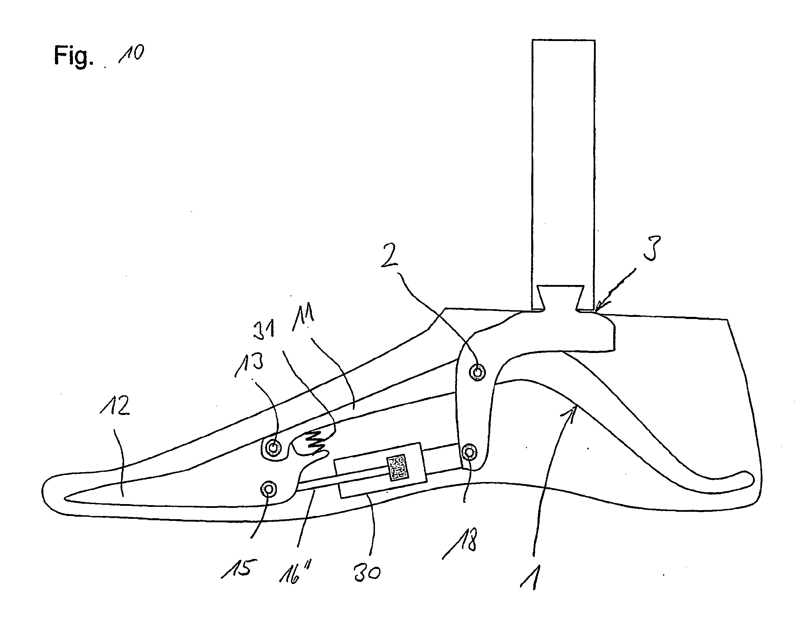

Figur 10 zeigt eine Ausführungsform des künstlichen Fußes, bei der der Lenker 16" mittels eines Hydraulikzylinders 30 längenveränderlich ausgebildet ist. Der Hydraulikzylinder 30 kann dabei, wie der Hydraulikzylinder 22 in Figur 7 bei einer kurzzeitigen Belastung eine Dämpfung der Übertragung der Winkelstellung des Anschlussteils 3 relativ zum Basis-Fußteil 1 auf die Winkelstellung des Vorfußteils 12 relativ zum Basis-Fußteil 1verzögern. Durch die Längenänderung des Hydraulikzylinders 30 kann dabei das Übersetzungsverhältnis der Parallelogrammführung zwischen dem Anschlussteil 3 und dem Vorfußteil 12 so geändert werden, dass ein übermäßiges Drücken des Fußteils 12 nach unten bei einer beim Abrollvorgang nach vorn geneigten Winkelstellung des Anschlussteils 3 relativ zum Basis-Fußteil vermieden wird. Eine direkte Übersetzung der Winkelstellung würde beim Abrollvorgang über das Vorfußteil 12 zu einem "Einkrallen" des Zehenbereichs des Vorfußteils 12 führen. Durch eine aufgrund der Längenänderung des Hydraulikzylinders 30 vorgenommene Änderung des Übersetzungsverhältnisses zwischen Anschlussteil 3 und Fußteil 12 kann dieser Einkralleffekt vermieden werden. Zwischen dem Vorfußteil 12 und dem Mittelfußabschnitt 11 des Basis-Fußteils 1 ist eine Feder 31 angeordnet, die eine Vorzugsstellung des Vorfußelements vorgibt. Die Feder 31 kann mit Vorteil durch eine Feder-Dämpfer-Kombination in einer Serienanordnung ersetzt werden.

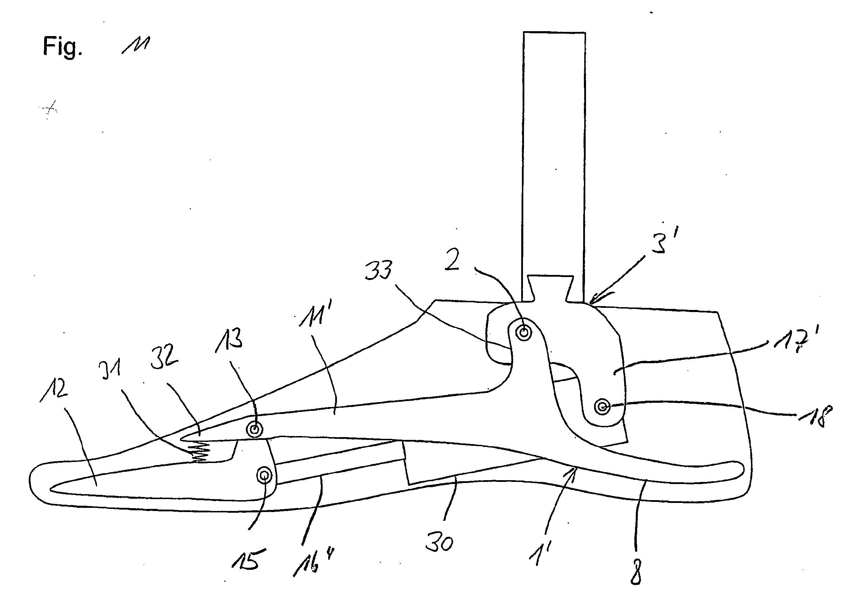

Figur 11 zeigt eine Ausführungsform, die mit der Ausführungsform gemäß Figur 10 gleichwirkend ausgeführt ist, jedoch konstruktive Modifikationen zeigt. So erstreckt sich das Basis-Fußteil 1' von dem Fersenbereich 8 nur leicht nach oben gewölbt in den Mittelfußteil 11' hinein. das Mittelfußteil 11' weist einen über das Drehgelenk 13 hinausragenden Ansatz 32 auf, der die auf das Vorfußteil 12 einwirkende Feder 31 bzw. die Feder-Dämpfer-Kombination trägt.

Das Anschlussteil 3' ist wiederum etwa L-förmig ausgebildet, jedoch ist der nach unten ragende Ansatz 17' nunmehr am posterioren Ende angeordnet, wodurch sich eine Rückverlagerung des Drehgelenks 18 in Richtung Fersenbereich ergibt. Dadurch steht für den Lenker 16" mit dem Hydraulik-Dämpfer 30 ein längerer Weg zur Verfügung.

Die Anlenkung des Basis-Fußteils 1' an das Knöchelgelenk 2 erfolgt über ein nach oben ragendes Ansatzstück 33 des Basis-Fußteils 1'.

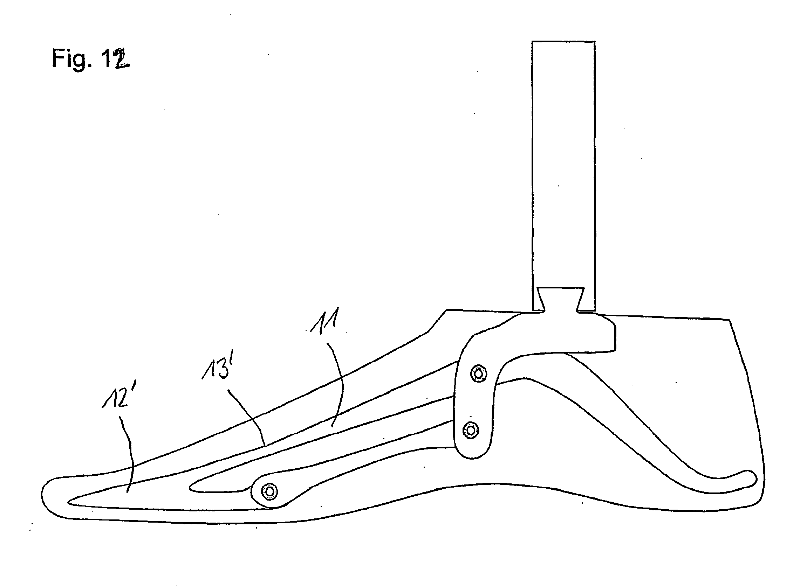

Die in Figur 12 dargestellte Ausführungsform entspricht im Wesentlichen der Ausführungsform gemäß Figur 1, wobei lediglich das Drehgelenk 13 entfallen und durch einen flexiblen Abschnitt 13' des Mittelfußabschnitts 11 ersetzt ist, der somit über den durch eine Materialverjüngung gebildeten flexiblen Abschnitt 13' in das Vorfußteil 12' übergeht. Selbstverständlich kann die Ausführungsform mit der Ausbildung des Gelenks 13' mit den in den übrigen Zeichnungsfiguren dargestellten Varianten kombiniert werden, also beispielsweise mit einem flexiblen Lenker 16' oder einem längenverstellbaren Lenker 16".

## Patentansprüche

1. Künstlicher Fuß mit einem Anschlussteil (3, 3') für ein Unterschenkelteil (5), einem mit dem Anschluss (3, 3') über ein Knöchelgelenk (2) drehbar verbundenen Basis-Fußteil (1, 1'), das sich posterior vom Knöchelgelenk (2) in einen Fersenabschnitt (8) und anterior vom Knöchelgelenk (2) in einen Mittelfußabschnitt (11, 11') erstreckt, und mit einem mit dem Mittelfußabschnitt (11, 11') drehbar verbundenen Vorfußteil (12, 12'), und einer Gelenkverbindung, die eine Winkelstellung des Anschlussteils (3, 3') relativ zum Basis-Fußteil (1, 1') proportional auf die Winkelstellung des Vorfußteils (12, 12') relativ zum Basis-Fußteil (1, 1') überträgt, **dadurch gekennzeichnet, dass** eine Veränderung der Winkelstellung des Anschlussteils (3, 3') nach posterior zu einem proportionalen Anheben des Vorfußteils (12, 12') führt.

2. Künstlicher Fuß nach Anspruch 1, **dadurch gekennzeichnet, dass** die Gelenkverbindung als Vier-Gelenkanordnung ausgebildet ist.

3. Künstlicher Fuß nach Anspruch 1 oder 2, **dadurch gekennzeichnet, dass** die Gelenkverbindung mit dem Mittelfußabschnitt (11, 11') und einem mit Vorfußteil (12, 12') und Anschlussteil (3, 3') gelenkig verbundenen Lenker (16, 16', 16") gebildet ist.

4. Künstlicher Fuß nach Anspruch 3, **dadurch gekennzeichnet, dass** der Mittelfußabschnitt (11, 11') und der Lenker (16, 16', 16") im Wesentlichen parallel zueinander angeordnet sind.

5. Künstlicher Fuß nach einem der Ansprüche 1 bis 4, **dadurch gekennzeichnet, dass** das Anschlussteil (3, 3') einen sich über das Knöchelgelenk (2) nach unten erstreckenden Ansatz (17, 17') aufweist, an dem der Lenker (16,16', 16") mit einem seiner Enden drehbar befestigt ist.

6. Künstlicher Fuß nach einem der Ansprüche 1 bis 5, **dadurch gekennzeichnet, dass** wenigstens eine gelenkige Verbindung durch einen flexiblen Abschnitt (13') eines Koppelstücks der Gelenkverbindung gebildet ist.

7. Künstlicher Fuß nach einem der Ansprüche 1 bis 6, **gekennzeichnet durch** ein Einstellelement (22) zwischen Basis-Fußteil (1) und Anschlussteil (3), dessen Verstellung erst bei einer über mehrere Sekunden andauernden Belastung wirksam wird.

8. Künstlicher Fuß nach Anspruch 7, **dadurch gekennzeichnet, dass** das Einstellelement (22) durch einen Hydraulikzylinder gebildet ist.

9. Künstlicher Fuß nach Anspruch 8, **dadurch gekennzeichnet, dass** in eine Umströmungsleitung (25), in der Hydraulikflüssigkeit von einer Seite auf die andere Seite eines Kolbens (24) des Hydraulikzylinders (22) strömt, eine Ventilanordnung (26) eingesetzt ist, die beim Überschreiten einer voreingestellten Strömungsgeschwindigkeit die Umströmungsleitung (25) sperrt.

10. Künstlicher Fuß nach einem der Ansprüche 7 bis 9, **dadurch gekennzeichnet, dass** das Einstellelement (22) mit einem durch kurzzeitige Belastungen verformbaren elastischen Glied kombiniert ist.

11. Künstlicher Fuß nach einem der Ansprüche 1 bis 10, **dadurch gekennzeichnet, dass** wenigstens ein Koppelstück (16') der Gelenkanordnung in Längsrichtung elastisch verformbar ausgebildet ist.

12. Künstlicher Fuß nach Anspruch 11, **dadurch gekennzeichnet, dass** das Koppelstück (16') in der Gelenkanordnung vorwiegend auf Zug belastet ist.

13. Künstlicher Fuß nach einem der Ansprüche 1 bis 12, **dadurch gekennzeichnet, dass** wenigstens ein Koppelstück (16") der Gelenkanordnung längenverstellbar ausgebildet ist.

14. Künstlicher Fuß nach Anspruch 13, **dadurch gekennzeichnet, dass** die Längenverstellung eines längenverstellbaren Koppelstücks (16') durch wenigstens einen Parameter der Geh- oder Stehsituation detektierenden Sensor steuerbar ist.

15. Künstlicher Fuß nach Anspruch 13 oder 14, **dadurch gekennzeichnet, dass** die Längenverstellung durch einen Hydraulikzylinder ausgeführt ist.

16. Künstlicher Fuß nach einem der Ansprüche 1 bis 15, **gekennzeichnet durch** ein elastisches Glied (31) zwischen Vorfußteil (12) und Basis-Fußteil (1, 1').

17. Künstlicher Fuß nach Anspruch 16, **gekennzeichnet durch** eine Feder-Dämpfer-Kombination zwischen Vorfußteil (12) und Basis-Fußteil (1, 1').

18. Künstlicher Fuß nach einem der Ansprüche 1 bis 17, **dadurch gekennzeichnet, dass** der Abstand der Gelenke (13, 13', 15) am Vorfußteil (12, 12') kleiner als der Abstand der Gelenke (2, 18) am Anschlussteil (3, 3') ist.

19. Künstlicher Fuß nach einem der Ansprüche 5 bis 18, **dadurch gekennzeichnet, dass** der Ansatz (17, 17') des Anschlussteils (3, 3') elastisch am Anschlussteil (3, 3') angebracht ist.

20. Künstlicher Fuß nach einem der Ansprüche 1 bis 19, **dadurch gekennzeichnet, dass** das Knöchelgelenk (2) mit Abstand anterior von einem Verbindungspunkt des Anschlussteils (3, 3') zum Unterschenkelteil (5) angeordnet ist.

21. Künstlicher Fuß nach einem der Ansprüche 1 bis 20, **dadurch gekennzeichnet, dass** das Basis-Fußteil (1) mit seinem Fersenabschnitt (8) von einem Sohlenbereich (14) ausgehend zum Anschlussteil (3) hin ansteigend ausgebildet ist, einen etwa geradlinig verlaufenden Mittelfußabschnitt (11) aufweist und mit dem Knöchelgelenk (2) bezüglich seiner axialen Länge mittig verbunden ist.

## Claims

1. An artificial foot with a connecting part (3, 3') for a lower leg part (5), with a base foot part (1, 1') which is pivotably connected to the connecting part (3, 3') via an ankle joint (2) and which extends rearward of the ankle joint (2) into a heel section (8) and forward of the ankle joint (2) into a midfoot section (11, 11'), and with a forefoot part (12, 12') pivotably connected to the midfoot section (11, 11'), and an articulated connection that transmits the angular position of the connecting part (3, 3') relative to the base foot part (1, 1') in a proportional manner to the angular position of the forefoot part (12, 12') relative to the base foot part (1, 1'), **characterized in that** a change in the angular position of the connecting part in the rearward direction leads to a proportional lifting of the forefoot part.

2. The artificial foot as claimed in claim 1, **characterized in that** the articulated connection is designed as a four-joint arrangement.

3. The artificial foot as claimed in claim 1 or 2, **characterized in that** the articulated connection is formed by the midfoot section (11, 11') and by a linking rod (16, 16', 16") that is connected in an articulated manner to forefoot part (12, 12') and connecting part (3, 3').

4. The artificial foot as claimed in claim 3, **characterized in that** the midfoot section (11, 11') and the linking rod (16, 16' 16") are arranged substantially parallel to one another.

5. The artificial foot as claimed in one of claims 1 through 4, **characterized in that** the connecting part (3, 3') has an extension piece (17, 17') which extends downward across the ankle joint (2) and on which the linking rod (16, 16', 16") is secured pivotably via one of its ends.

6. The artificial foot as claimed in one of claims 1 through 5, **characterized in that** at least one hinged connection is formed by a flexible section (13') of a coupling piece of the articulated connection.

7. The artificial foot as claimed in one of claims 1 through 6, **characterized by** an adjustment element (22) which is arranged between base foot part (1) and connecting part (3) and whose adjusting action comes into effect only when there is a load that continues over several seconds.

8. The artificial foot as claimed in claim 7, **characterized in that** the adjustment element (22) is formed by a hydraulic cylinder.

9. The artificial foot as claimed in claim 8, **characterized in that** a valve arrangement (26) is inserted into a circuit line (25) in which hydraulic fluid flows from one side to the other side of a piston (24) of the hydraulic cylinder (22), which valve arrangement (26) blocks the circuit line (25) when a predetermined flow velocity is exceeded.

10. The artificial foot as claimed in one of claims 7 through 9, **characterized in that** the adjustment element (22) is combined with an elastic member that can be deformed by momentary loads.

11. The artificial foot as claimed in one of claims 1 through 10, **characterized in that** at least one coupling piece (16') of the articulated arrangement is designed to be elastically deformable in the longitudinal direction.

12. The artificial foot as claimed in claim 11, **characterized in that** the coupling piece (16') in the articulated arrangement is subjected mainly to tensile loading.

13. The artificial foot as claimed in one of claims 1 through 12, **characterized in that** at least one coupling piece (16") of the articulated arrangement is designed to be adjustable in length.

14. The artificial foot as claimed in claim 13, **characterized in that** the length adjustment of a length-adjustable coupling piece (16') can be controlled by at least one sensor that detects parameters of the walking or standing situation.

15. The artificial foot as claimed in claim 13 or 14, **characterized in that** the length adjustment is performed by a hydraulic cylinder.

16. The artificial foot as claimed in one of claims 1 through 15, **characterized by** an elastic member (31) between forefoot part (12) and base foot part (1, 1').

17. The artificial foot as claimed in claim 16, **characterized by** a spring/damper combination between forefoot part (12) and base foot part (1, 1').

18. The artificial foot as claimed in one of claims 1 through 17, **characterized in that** the spacing between the joints (13, 13', 15) on the forefoot part (12, 12') is smaller than the spacing between the joints (2, 18) on the connecting part (3, 3').

19. The artificial foot as claimed in one of claims 5 through 18, **characterized in that** the extension piece (17, 17') of the connecting part (3, 3') is mounted elastically on the connecting part (3, 3').

20. The artificial foot as claimed in one of claims 1 through 19, **characterized in that** the ankle joint (2) is arranged at a distance forward of a connection point between the connecting part (3, 3') and the lower leg part (5).

21. The artificial foot as claimed in one of claims 1 through 20, **characterized in that**, starting from a sole area (14), the heel section (8) of the base foot part (1) is designed ascending toward the connecting part (3), comprises an approximately rectilinear midfoot section (11), and is connected to the ankle joint (2) centrally in relation to its axial length.

## Revendications

1. Pied artificiel avec une partie de raccordement (3, 3') pour une partie de jambe (5), une partie de base de pied (1, 1') reliée à la partie de raccordement (3, 3') via une articulation de cheville (2), ladite partie de base s'étendant en direction postérieure depuis l'articulation de cheville (2) dans un tronçon de talon (8) et en direction antérieure depuis l'articulation de cheville (2) dans un tronçon de pied médian (11, 11'), et comprenant une partie d'avant-pied (12, 12') reliée avec possibilité de rotation au tronçon de pied médian (11, 11'), et une liaison articulée qui transmet une position angulaire de la partie de raccordement (3, 3') de manière proportionnelle à la position angulaire de la partie d'avant-pied (12, 12') par rapport à la partie de base de pied (1, 1'), **caractérisé en ce qu'**une modification de la position angulaire de la partie de raccordement (3, 3') en direction postérieure mène à un soulèvement proportionnel de la partie d'avant-pied (12, 12').

2. Pied artificiel selon la revendication 1, **caractérisé en ce que** la liaison articulée est réalisée sous la forme d'un agencement à quadrilatère articulé.

3. Pied artificiel selon la revendication 1 ou 2, **caractérisé en ce que** la liaison articulée est formée avec le tronçon de pied médian (11, 11') et avec un bras (16, 16', 16") relié de manière articulée avec la partie d'avant-pied (12, 12') et avec la partie de raccordement (3, 3').

4. Pied artificiel selon la revendication 3, **caractérisé en ce que** le tronçon de pied médian (11, 11') et le bras (16, 16', 16") sont agencés sensiblement parallèlement l'un à l'autre.

5. Pied artificiel selon l'une des revendications 1 à 4, **caractérisé en ce que** la partie de raccordement (3, 3') comprend un prolongement (17, 17') qui s'étend vers le bas au-delà de l'articulation de cheville (2) et auquel le bras (16, 16', 16") est fixé par l'une de ses extrémités avec possibilité de rotation.

6. Pied artificiel selon l'une des revendications 1 à 5, **caractérisé en ce qu'**au moins une liaison avec articulation est formée par un tronçon flexible (13') d'une pièce d'accouplement de la liaison articulée.

7. Pied artificiel selon l'une des revendications 1 à 6, **caractérisé par** un élément de réglage (22) entre la partie de base de pied (A) et la partie de raccordement (3), dont le réglage devient actif uniquement en cas d'une charge durant plus de plusieurs secondes.

8. Pied artificiel selon la revendication 7, **caractérisé en ce que** l'élément de réglage (22) est formé par un cylindre hydraulique.

9. Pied artificiel selon la revendication 8, **caractérisé en ce qu'**un agencement de valve (26) est intégré dans une conduite de dérivation (25), dans laquelle un liquide hydraulique s'écoule depuis à côté d'un piston (24) du cylindre hydraulique (22) vers l'autre côté, l'agencement de valve fermant la conduite de dérivation (25) en cas de dépassement d'une vitesse d'écoulement préétablie.

10. Pied artificiel selon l'une des revendications 7 à 9, **caractérisé en ce que** l'élément de réglage (22) est combiné avec un organe élastique déformable par des charges de brève durée.

11. Pied artificiel selon l'une des revendications 1 à 10, **caractérisé en ce qu'**au moins une pièce d'accouplement (16') de l'agencement articulé est réalisée élastiquement déformable en direction longitudinale.

12. Pied artificiel selon la revendication 11, **caractérisé en ce que** la pièce d'accouplement (16') dans l'agencement articulé est chargée principalement en traction.

13. Pied artificiel selon l'une des revendications 1 à 12, **caractérisé en ce qu'**au moins une pièce d'accouplement (16") de l'agencement articulé est réalisée avec possibilité de réglage en longueur.

14. Pied artificiel selon la revendication raison, **caractérisé en ce que** le réglage en longueur d'une pièce d'accouplement réglable en longueur (16') est susceptible d'être commandé par au moins un paramètre du capteur qui détecte la situation de marche ou de station debout.

15. Pied artificiel selon la revendication 13 ou 14, **caractérisé en ce que** le réglage en longueur est exécuté par un cylindre hydraulique.

16. Pied artificiel selon l'une des revendications 1 à 15, **caractérisé par** un organe élastique (31) entre la partie d'avant-pied (12) et la partie de base de pied (1, 1').

17. Pied artificiel selon la revendication 16, **caractérisé par** une combinaison ressort/amortisseur entre la partie d'avant-pied (12) et la partie de base de pied (1, 1').

18. Pied artificiel selon l'une des revendications 1 à 17, **caractérisé en ce que** la distance des articulations (13, 13', 15) sur la partie d'avant-pied (12, 12') est plus petite que la distance des articulations (2, 18) sur la partie de raccordement (3, 3').

19. Pied artificiel selon l'une des revendications 5 à 18, **caractérisé en ce que** le prolongement (17, 17') de la partie de raccordement (3, 3') est rapporté de manière élastique sur la partie de raccordement (3, 3').

20. Pied artificiel selon l'une des revendications 1 à 19, **caractérisé en ce que** l'articulation de cheville (2) est agencée à distance en direction antérieure depuis un point de liaison de la partie de raccordement (3, 3') vers la partie de jambe (5).

21. Pied artificiel selon l'une des revendications 1 à 20, **caractérisé en ce que** la partie de base de pied (1) avec son tronçon de talon (8) est réalisée de manière à monter en partant d'une zone de semelle (14) en direction de la partie de raccordement (3), comporte un tronçon de pied médian (11) qui s'étend environ en ligne droite, et est reliée à l'articulation de cheville (2) au milieu par référence à sa longueur axiale.
